# EUROPEAN PATENT SPECIFICATION

(11) **EP 0 203 649 B1**
(45) Date of publication and mention of the grant of the patent: **02.08.1995**
(21) Application number: 86200822.4
(22) Date of filing: 13.05.1986
(51) Int. Cl.: A61K 31/66

(54) **Pharmaceutic composition based on diphoshonates for the treatment of the arthrosis**
Diphosphonate enthaltende pharmazeutische Zusammensetzungen zur Behandlung von Arthrosis
Compositions pharmaceutiques contenant des diphosphonates pour le traitement de l'arthrose

(30) Priority: 24.05.1985 IT 6747885
(43) Date of publication of application: 03.12.1986
(73) Proprietor: ISTITUTO GENTILI S.p.A., 56125 Pisa (IT)
(72) Inventor: Rosini, Sergio, I-57100 Ardenza(Livorno) (IT); Fontanelli, Luciano, I-56100 Pisa (IT)
(74) Representative: Minoja, Fabrizio

(56) References cited:
- EP-A- 0 088 462
- EP-A- 0 094 714
- CLINICAL ORTHOPAEDICS AND RELATED RESEARCH, VOL. 155, MARCH-APRIL 1981, pp. 218-223; M. WALTON: "The effects of longterm administration of ethane-1-hydroxy-1,1-diphosphonate on osteo-arthrosisand heterotropic ossification in the mouse knee joint"
- MSD Res. Labs., Rahway, N.J., US, 14th ed., 1982; R. BERKOW et al.:"THE MERK MANUAL OF DIAGNOSIS AND THERAPY"
- CURRENT RESEARCH AND PROSPECTS FOR PHARMACOLOGICAL INTERVENTION, IBC Technical Services Ltd; R.G.G. RUSSELL: "OSTEOARTHRITES"

## Description

It is known that arthrosis is a degenerative pathogenesis event which involves the articular structures.

While in the past arthrosis was considered a senility phenomenon, i.e. due to the ageing of the articular structures, nowadays, though granting the existence of a senile variant, comprising the forms of secondary arthrosis connected to a pathology of the ageing, clinical observation allowed to identify also cases of appearance even at a relatively young age.

Thus, a distinction has been created between primary arthrosis connected to an intrinsic disease of the articular structures and secondary arthrosis connected to factors which are extrinsic with respect to the articular structure itself.

All authors agree in considering arthrosis to be an extremely diffused disease, certainly the most common among those which injure the joints; its frequency increases with age to such an extent that nobody avoids it.

Morphological researches have proved that the first alterations interest the surface cartilagenous layers (in the zone of the joints) with a depolymerization and a flight of cartilagenous mucopolysaccharides. These latter are accompanied by physicochemical alterations of the collagenous fibres which become less resistant and finally break the front of resistance of the cartilage. Such an event is accompanied by a chondrocitary reaction which denotes an attempt of compensation for the reconstruction of the altered structures.

It may however be said that the main pathogenetic event of the arthrosis is represented by mechanisms which give rise to the cartilagenous injury. In fact, it has been ascertained that the various etiological factors are able, after having exceeded a certain damaging threshold, to liberate a series of substances which are noxious to the articular structures.

The liberation of mediators may directly induce a damaging action or activate the liberation of other mediators, mostly degenerative enzymes, contained in the cartilagenous and sinovial structures.

On this basis also a phlogistic event, as specific and secondary reaction, with the intervention of polynucleates and the liberation of the articular cavity from their lithic mediators which can act both directly and by activating the prostaglandinic systems.

As a consequence of the foregoing, extremely disabling situations arise, accompanied by highly painful symptomatologies which may be classified on the basis of the degree of the existing alterations.

The main evaluation means are given by the inhibition of the movement, the radiographic data, the composition of the sinovial liquid and, in recent times the limboscopic reports.

The therapeutic means used until today have proved to be clearly insufficient to treat such disease, and the substances used, such as the analgesics, the non-steroidal antiphlogistics, the cortisonics, besides being simply symptomatic, have proved to create serious side damaging effects

The present invention refers to the use of a diphosphonate selected from:
a) a diphosphonate of the formula 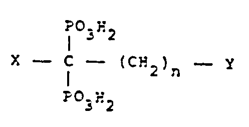 wherein X is OH, Cl F or H
   Y is NH₂, Cl, F H
   and n is different from 1 and when X is OH and Y is NH₂, n is different from 2;
b) clodronic acid or difluoromethane diphosphonic acid; for the preparation of a medicament to be administered intra-articularly for the treatment of osteoarthritis, said medicament consisting of an aqueous solution, having a pH value from 4.5 to 7.5 and a diphosphonate concentration from 10⁻¹ to 10⁻⁶ M.

According to one of its aspects, this invention consists, for example, of a composition comprising an efficient and non-toxic dose of sodium chlodronate in a suitable solvent to be administered by intraarticular way.

The sodium chlodronate and the others diphosphonates mentioned hereinabove represent a category of known substances which are used up to today for the prevention and the treatment of certain pathological situations in animals and in men, connected with an abnormal metabolism of the calcium and the phosphate.

According to the known technique, such situations include the osteoporosis, the Paget's disease, the ossifying myositis, the universal calcinosis, the artherosclerosis and other situations which identify the inducing event in the altered mobilization or deposition of the calcium. To this end the diphosphonates have been utilized hitherto for oral, intramuscular and intravenous administrations, sometimes in extremely high dosages up to 500 mg by intravenous route and 2 - 3 g by oral route daily, with non-negligible side effects.

Clinical Orthop. & Related Res., no.155, 218-223, 1981 reports about the effects of long term i.p. administration of ethane-1-hydroxy-1,1-diphosphonate on osteoarthrosis in the mouse knee joint. No significant therapeutic effect was found. EP-A-0088462 discloses combination of steroids and diphosphonates for the treatment of rheumatoid arthritis. EP-A-94714 discloses intraarticular injection of 1-hydroxy 3 aminopropane diphosphonic acid

According to this invention the Applicant has had the opportunity to observe and clinically prove that the sodium chlodronate may surprisingly be used in the treatment of the arthrosis by employing a new way of intraarticular administration and extremely low and non-toxic concentrations of the product.

The mechanisms of action which may be supposed to exist are humoral effects (inhibition of the prostaglandines, of the lactate syntheses) and cellular effects (increase in the synthesis of the proteoglycanes).

According to the invention, the necessary doses of sodium chlodronate or of the other diphosphonates proposed, may obviously vary according to the seriousness of the situation, the advisable duration of the treatment , as well as the various types of the diphosphonate used.

Generally, concentrations of sodium chlodronate between 1 and 100 »g (γ) for each administration are suitable, as well as the dosages of the sodium etidronate and the hydroxypentandiphosphonate.

In the case of aminoderivatives, in consideration of their higher activity and toxicity, it is necessary to use reduced dosages comprised between 0,01 and 10 »g (γ) each administration.

The intraarticular administration is carried out by using particular preparations. To this end, aqueous solutions of the alkylidene-diphosphonic acids or their salts (of alkaline-earth or alkaline metals, preferably sodium) at concentrations variable between 10⁻¹ and 10⁻⁶ M, with pH comprised between 4.5 and 7.5, have been prepared initially and isotonized by adding sodium chloride and other suitable excipients (preferably glucose); in some cases, preservatives (preferably methyl-p-hydroxy-benzoate) and analgesic agents (preferably lidocaine hydrochloride) have also been added.

Some of the solutions have been introduced into phials as such whilst others have been mixed with support substances (preferably glycine or lisine) in order to lyophilize them.

Subsequently, samples of all the solutions (including those to be lyophilized) have been subjected to accelerated stability tests (GARRETT E.R., J.A.P.A.S., 44, 515), and in accordance with what has been stated in the literature for various diphosphonic acids, (Bikman B.I., URINOVICH E.M. and Coll. -, Zh Neorg. Khim 1973, 18 (9), 2406-9; -Burton D.J. Pietrzyk D.J. and Coll., J. Fluorine Chem. 1982, 20 (5) 61726.) all the tested alkylidene-diphosphonic acids have proved to be perfectly thermostable.

After months of storage at ambient temperature and sheltered from the light,the solutions as such introduced into phials have evidenced the presence of bodies on the bottom, and especially those which have lower, concentrations even though their titre was generally corresponding. We have however noticed that no negative action on the stability of the solutions could be ascribed to the p-hydroxy-benzoates, the buffers and the isotonizing agents, whose action , instead, seemed to be connected to the different concentrations and kind of the diphosphonic acids, in as much as with some of them, for example with ethane-1-hydroxy-1, 1-diphosphonic (EHDP) acid, the characteristics of instability do not appear.

All the solutions have instead proved to be suprisingly free from defects at any concentration of the various alkilidene-diphosphonic acids used, which have been prepared with the addition of aminoacids as support substances for the lyophilization.

We have therefore prepared new solutions containing variable quantities of amino-acids (preferably glycine or lysine) as stabilizers, and have ascertained the inalterability, as the time passes, of such liquid preparations.

In this way it has thus been discovered the means for obtaining liquid formulations in which no precipitations take place and which, by suitably dosing the quantity of amino-acids, may be lyophilized.

Some pharmaceutical formulations suitable to be used by intraarticular route, which illustrate said discovery, are described in the following examples.

### EXAMPLE 1 : Sodium chlodronate solution for intraarticular use.

| | |
|---|---|
| Sodium chlodronate | mg 1,000 |
| 10% acetic acid | mg 2,250 |
| Anhydrous sodium hydroxide | mg 1,520 |
| Glycine | mg 15,000 |
| Purified water,quantity sufficient to obtain | ml 1,000 |
| pH 5,65 | |

Compositions substantially identical to those of Example 1 may be prepared also with the salts of 3-amino-1-hydroxypropylidene-1, 1-diphosphonic (AHPrBP), 4-amino-1-hydroxybutylidene-1, 1-diphosphonic (AHBuBP) 5-amino-1-hydroxypentylidene-1, 1-diphosphonic (AHPeBP) and 6-amino-1-hydroxyhexylidene-1, 1-diphosphonic (AHEBP) acids and the difluoromethylenediphosphonic (Fl₂MBP) acid.

The concentrations of the active principle may range between 10 and 1000 γ.

### EXAMPLE II : Sodium chlodronate solution for peri-articular use.

| | |
|---|---|
| Sodium chlodronate | mg 1,000 |
| Anhydrous sodium hydroxide | mg 0,325 |
| Lidocaine hydrochloride | mg 1,000 |
| Glycine | mg 20,000 |
| Water for injectable elements, quantity sufficient to obtain | ml 1,000 |
| pH 6,45 | |

Various solutions similar to those of the solution of Example II may be prepared using the acids AHPrBP, AHBuBP, AHPeBP, AHEBP instead of chlodronic acid.

The concentrations may range between 1000 and 10 γ/ml for all the acids, including the chlodronic acid.

### EXAMPLE III : Solution for peri- and intraarticular use, presented in a liophilized bottle (A) + a phial of solvent (B)

### A - Lyophilized bottle

| | |
|---|---|
| Sodium chlodronate | »g 50,00 |
| Glycine | mg 125,00 |
| Procaine hydrochloride | mg 10,00 |
| Water, a quantity sufficient to obtain | ml 0,50 |
| pH 6,0 | |
| Solution (A) is subjected to lyophilization. | |

### B - Phial of solvent

| | |
|---|---|
| Methyl p-hydroxybenzoate | mg 2,500 |
| Anhydrous acetic acid | mg 2,250 |
| Anhydrous sodium hydroxide | mg 1,525 |
| Water for injectable elements, a quantity sufficient to obtain | ml 5,000 |
| pH 6,0 | |

Compositions substantially corresponding to those indicated in "A" may be obtained by substituting the sodium chlodronate by a sodium salt of AHPrBP, AHPeBP,AHEBP, Fl₂MBP and, generally, of any diphosphonic acid.

The concentrations of such acids (as well as that of the chlodronic acid) may vary between 0,1 and 1000 without the need of varying any excipient.

The efficiency of the formulations provided by the present invention for the treatment of the arthrosis has been proved by an accurate research carried out in hospital conditions.

The patients admitted to the experimentation were men and women, aged between 65 and 80 years, with a serious form of arthrosis of the ilium or the knee or of the scapulo-humeral region in a pre-degenerative stage, and for whom, due to the lack of other valuable therapeutic means, a surgical operation has been suggested.

The stage of the affections was evaluated by observing the tumefaction, the alterations of the sinovial liquid, the degree of the motorial limitations and the radiographic reports directed to the articular rima and to the limboscopic report carried out by a complex methodology capable of providing also photographic images.

The patients subjected to the research were excluded from any other type of treatment and were treated with disphosphonates by intraarticular route with doses comprised between 10 and 100 »g in a volume from 0,5 to 2 ml. The above and other parameters concerning the tolerability were evaluated at the time 0 and than after 7-10 days and also after 14-20 days at the second and third administration of the medicine.

Surprisingly, the analyses carried out have proved that about 10% of the patients have obtained a striking improvement with a normalization of the objective parameters and a return to an almost normal articular functionality when considering the age of the patients. Among the patients who have received only a partial benefit, the repetition of the cycle of treatment and the administration of the compound by intramuscular way with daily doses of 100 mg, or at alternate days, for 15-30 days have led to a further increase of the percentage of the patients whose conditions have improved.

In all cases the tolerability of the medicament was perfect and without any modification of the bihumoral parameters which were examined.

## Claims

1. The use of a diphosphonate selected from:
a) a diphosphonate of the formula 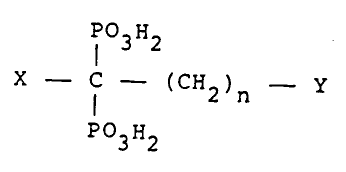 wherein X is OH, Cl F or H
Y is NH₂, Cl, F H
and n is from 1 to 5 with the proviso that when Y = NH₂ n is different from 1 and when X is OH and Y is NH₂, n is different from 2;
b) clodronic acid or difluoromethane diphosphonic acid;
for the preparation of a medicament to be administered intra-articularly for the treatment of osteoarthritis, said medicament consisting of an aqueous solution, having a pH value from 4.5 to 7.5 and a diphosphonate concentration from 10-¹ to 10⁻⁶ M.

2. The use according to claim 1 wherein the diphosphonate is selected from dichloromethylene diphosphonic acid, ethane-1-hydroxyl-1,1-diphosphonic acid, difluoromethane diphosphonic acid, pentane-1-hydroxy-1,1-diphosphonlc acid, 3-amino-1-hydroxypropylidene-1,1-diphosphonic acid, 4-amino-1-hydroxybutylidene-1,1-diphosphonic acid, 5-amino-1-hydroxypentylidene-1,1-diphosphonic acid, 6-amino-1-hydroxyhexylidene-1,1-diphosphonic acid or pharmaceutically acceptable salts thereof.

3. The use according to claim 1 or 2 wherein aminoacids are used in the preparation of the medicament as stabilizers thereof.

4. The use according to claim 3 wherein the aminoacids are glycine, lysine or alanine.

## Patentansprüche

1. Verwendung eines Diphosphonats, ausgewählt aus:
a) einem Diphosphonat der Formel 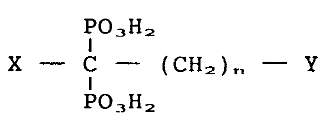 worin X für OH, Cl, F oder H steht,
Y für NH₂, Cl, F oder H steht
und n den Wert 1 bis 5 hat, mit der Maßgabe, daß, wenn Y = NH₂, n von 1 verschieden ist, und daß, wenn X für OH und Y für NH₂ steht, n von 2 verschieden ist;
b) Clodronsäure oder Difluormethandiphosphonsäure;
zur Herstellung eines intraartikulär zur Behandlung von Osteoarthritis zu verabreichenden Arzneimittels, wobei das Arzneimittel aus einer wäßrigen Lösung besteht, die einen pH-Wert von 4,5 bis 7,5 und eine Diphosphonatkonzentration von 10⁻¹ bis 10⁻⁶ M hat.

2. Verwendung nach Anspruch 1, dadurch **gekennzeichnet,** daß das Diphosphonat aus Dichlormethylendiphosphonsäure, Ethan-1-hydroxy-1,1-diphosphonsäure, Difluormethandiphosphonsäure, Pentan-1-hydroxy-1,1-diphosphonsäure, 3-Amino-1-hydroxypropyliden-1,1-diphosphonsäure, 4-Amino-1-hydroxybutyliden-1,1-diphosphonsäure, 5-Amino-1-hydroxypentyliden-1,1-diphosphonsäure, 6-Amino-1-hydroxyhexyliden-1,1-diphosphonsäure oder pharmazeutisch annehmbaren Salzen davon ausgewählt ist.

3. Verwendung nach Anspruch 1 oder 2, dadurch **gekennzeichnet,** daß Aminosäuren zur Herstellung des Medikaments als Stabilisatoren hierfür verwendet worden sind.

4. Verwendung nach Anspruch 3, dadurch **gekennzeichnet,** daß die Aminosäuren Glycin, Lysin oder Alanin sind.

## Revendications

1. Utilisation d'un diphosphonate choisi entre :
a) un diphosphonate de formule 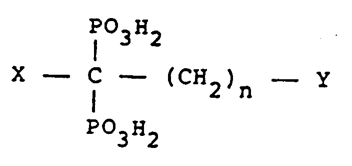 dans laquelle X est OH, Cl, F ou H
Y est NH₂, Cl, F ou H
et n vaut de 1 à 5, avec la réserve que lorsque Y = NH₂, n est différent de 1 et lorsque X est OH et Y est NH₂, n est différent de 2 ;
b) l'acide clodronique ou l'acide difluorométhane diphosphonique ;
pour la préparation d'un médicament à administrer par voie intra-articulaire pour le traitement de l'ostéoarthrite, ledit médicament étant constitué d'une solution aqueuse ayant un pH de 4,5 à 7,5 et une concentration en diphosphonate de 10⁻¹ à 10⁻⁶M.

2. Utilisation selon la revendication 1, dans laquelle le diphosphonate est choisi entre l'acide dichlorométhylène diphosphonique, l'acide éthane-1-hydroxy-1,1-diphosphonique, l'acide difluorométhane diphosphonique, l'acide pentane-1-hydroxy-1,1-diphosphonique, l'acide 3-amino-1-hydroxypropylidène-1,1-diphosphonique, l'acide 4-amino-1-hydroxybutylidène-1,1-diphosphonique, l'acide 5-amino-1-hydroxypentylidène-1,1-diphosphonique, l'acide 6-amino-1-hydroxyhexylidène-1,1-diphosphonique et leurs sels pharmaceutiquement acceptables.

3. Utilisation selon la revendication 1 ou 2, dans laquelle des acides aminés sont utilisés dans la préparation du médicament en tant qu'agents stabilisants de celui-ci.

4. Utilisation selon la revendication 3, dans laquelle les acides aminés sont la glycine, la lysine et l'alanine.
